**Europäisches Patentamt**

**European Patent Office**

**Office européen des brevets**

(11) Publication number: **0 314 040 B1**

# EUROPEAN PATENT SPECIFICATION

(45) Date of publication of patent specification: **08.03.95**

(21) Application number: **88117670.5**

(22) Date of filing: **24.10.88**

(51) Int. Cl.6: **B01D 53/04**, C10K 1/32, C07C 7/12, C07C 9/04, C01B 3/56

(54) **Method for removing carbon dioxide gas and moisture in a city gas production.**

(30) Priority: **24.10.87 JP 269916/87**

(43) Date of publication of application:
**03.05.89 Bulletin 89/18**

(45) Publication of the grant of the patent:
**08.03.95 Bulletin 95/10**

(84) Designated Contracting States:
**DE FR GB**

(56) References cited:
**EP-A- 0 193 716**
**DE-A- 2 624 346**
**GB-A- 2 155 805**
**US-A- 4 404 004**
**US-A- 4 705 541**

(73) Proprietor: **SAIBU GAS CO.,LTD.**
**13-20, Kego 2-chome**
**Chuo-ku**
**Fukuoka-shi**
**Fukuoka-ken (JP)**

(72) Inventor: **Kanamaru, Toshihisa**
**51-6, Sunny Town**
**Kashii**
**Higashi-ku**
**Fukuoka-shi**
**Fukuoka-ken (JP)**
Inventor: **Urano, Shoji**
**26-15, Miyake 1-chome**
**Minami-ku**
**Fukuoka-shi**
**Fukuoka-ken (JP)**
Inventor: **Ohta, Kei**
**31-16, Miwadai 1-chome**
**Higashi-ku**
**Fukuoka-shi**
**Fukuoka-ken (JP)**
Inventor: **Kawasaki, Shunji**
**12-437, Akenono 1-chome**
**Sawara-ku**
**Fukuoka-shi**
**Fukuoka-ken (JP)**

(74) Representative: **Ritter und Edler von Fischern, Bernhard,Dipl.-Ing. et al Hoffmann, Eitle & Partner, Patentanwälte, Postfach 81 04 20 D-81904 München (DE)**

**Description**

This invention relates to a refinement of reformed gas in a process for producing a city gas, more particularly a method for removing carbon dioxide gas as an incombustible component and separating moisture which is harmful in corroding gas pipe lines and the like.

Conventionally, in case of production of city gas with high calorific value which is produced as a substitute to natural gas (i.e. SNG), as a means for producing city gas, generally mixed gases containing such as $CH_4$, $H_2$, CO, $CO_2$, $N_2$ and $H_2O$ are produced in a gas producing device firstly, and secondly those unnecessary contents such as $CO_2$ and $H_2O$ within the mixed gases are removed and separated, and then lastly the remainders are regulated in calorific value and combustion characteristic by adding butane.

As to the removal and separation of $CO_2$ gas and $H_2O$ moisture, conventionally they are treated by those two processes separately such as in a wet type carbon dioxide gas removing device in which the carbon dioxide gas is absorbed by an absorption liquid such as potassium carbonate and the like, on the other hand, in a dehydrating device for separating a condensed water. In these cases, however, it has such disadvantages that it takes a lot of time until the said processes go into a normal operation state from the start up and load fluctuations cause operation complications. Therefore, it has been desired to solve these problems on operation and economical efficiency as for the removal and separation of $CO_2$ gas and $H_2O$ moisture. Conventionally, the pressure swing adsorption method has been adopted, that is, those two mechanisms for the removal and the separation of carbon dioxide gas and moisture are incorporated within one unit wherein the device itself and the operation become comparatively simplified and easier.

In case of conventional city gas production however, it is not only required to remove and separate the carbon dioxide gas and the moisture but also the gas producing device to be upstream and the calorific value regulating device to be downstream should be corresponded with the continuous stream in the whole process, whereby the pressure swing adsorption method is deemed to be disadvantageous because of those fluctuations of flow rate, pressure and calorific value, thus a steady state is hard to keep over the whole process. Further in the pressure swing adsorption method and in the prior technology, the exhaust gas (i.e. rest gas) which contains a lot of combustible components are by-produced, consequently the treatment of said exhaust gas involves many difficulties, particularly as a most unfavorable defect, it comes into question that it causes the loss of available components for the city gas. This is the reason why the pressure swing adsorption method has not been adopted as a useful method in the city gas production so far.

EP-A-0 193 716 describes a process for the adsorptive separation of a gas mixture containing at least a primary gaseous component and a secondary gaseous component in which each component is recovered at high recovery and high purity in a plurality of adsorption beds which are preferentially selective to the adsorption of the secondary component, comprising the steps of:

(a) passing a feed gas mixture into one of the plurality of adsorption beds wherein primary component passes through the bed as a pure product and secondary component is selectively adsorbed on the adsorbent;

(b) discontinuing adsorption and rinsing the adsorption bed with a stream of secondary component to remove any primary component as an effluent which is recycled to the feed to the process;

(c) depressurizing the rinsed adsorption bed to an intermediate pressure countercurrent to said adsorption to at least partially remove the secondary component from said adsorption bed;

(d) following said depressurization of step (c) and without further rinsing, evacuating said adsorption bed countercurrently to a subatmospheric pressure to further remove secondary component from said bed; and

(e) repressurizing the bed to superatmospheric pressure to prepare the bed for another adsorption step.

The object of the present invention is to recover the available components (combustible gases) as much as possible toward the city gas which are contained within the exhaust gas produced by the pressure swing adsorption method as first, that is to say, any losses accompanied with aforesaid processes of removal and separation are to be reduced as little as possible, and secondly those fluctuations of flow rate, pressure and calorific value are to be eliminated in the prior art and after sequential processing.

The object of the present invention is solved by a method for continuously removing carbon dioxide gas and water moisture from a reformed gas produced in the process of city gas production; the method comprising a pressure swing adsorption process utilizing at least four adsorption columns containing carbon molecular sieves with an average pore diameter of 0.3 nm (3A) as adsorbent, each of which successively undergoing a cyclical manner the steps of:

(a) feeding at an elevated pressure the wet reformed gas without any water content reducing pretreatment into the inlet of the adsorption column, adsorbing carbon dioxide and water from gas and

EP 0 314 040 B1

withdrawing refined gas from the outlet of the adsorption column;

(b) readsorbing at elevated pressure carbon dioxide from a gas stream which is concurrently fed from a water-sealed gas holder into the adsorption column and recycling the gas which is withdrawn from the outlet of the adsorption column during this step to the wet reformed gas feed stream;

(c) equalizing the gas pressure between said adsorption column and a parallel column which has terminated the evacuation step (f) by bringing in fluid communication respectively the inlets and the outlets of both columns;

(d) countercurrently further depressurizing the adsorption column by bringing its inlet in fluid communication to the water sealed gas holder, wherein the pressure of this gas is regulated;

(e) further countercurrently depressurizing the adsorption column by mechanical suction and introducing the gas which is sucked off into the water-sealed gas holder, as well;

(f) further countercurrently depressurizing the adsorption column by mechanical suction to desorb and extract carbon dioxide and water from the adsorbent and discarding the gas which is sucked off;

(g) primarily repressurizing said adsorption column by equalizing the gas pressure between said adsorption column and another column which has terminated the readsorption step (b) and bringing therefor in fluid communication respectively the inlets and the outlet of both columns and simultaneously secondarily repressurizing said adsorption column by countercurrently introducing a partial amount of the refined gas stream via a constant flow rate controlling valve; and

(h) further secondarily repressurizing said adsorption column towards the pressure of the adsorption step (a) by countercurrently introducing a partial amount of the refined gas stream via a constant flow rate controlling valve.

In the following the invention is described in more detail with reference to the drawings, in which:

Fig. 1 shows a schematic flow sheet showing a summarized construction of a device for practicing a process for removing and separating those of carbon dioxide gas and moisture in a process for producing a city gas under the present invention.

Fig. 2 shows a flow sheet for explaining a principle of process in the present invention.

Fig. 3 shows a graph showing a pressure variation within adsorption columns upon each process and step in a method provided by the present invention and

Fig. 4 shows a block diagram showing each process and those columns which are given in parentheses show their working columns respectively when column A is in the state of refining (adsorption).

In this invention, the reformed gas (hereinafter it is referred to as reformed gas prior to processing) consists mainly of combustible components such as $CH_4$ and $H_2$ which are produced in a process of city gas production from methanol as a raw material for example, which is generally a wet type mixed gas containing $CO_2$ gas and moisture, and then the reformed gas prior processed is supplied from the gas producing device under a continuously constant pressure with a predetermined flow rate.

As to the feed pressure of reformed gas prior to processing, it is determined in accordance with the reaction pressure within the gas producing device, however, it will be 0,88 MPa (9Kg/cm$^2$) in this case hereinafter.

That is, the reformed gas prior to processing is fed into the adsorption columns of the pressure swing adsorption device (hereinafter it is referred to as PSA device) wherein $CO_2$ gas and $H_2O$ moisture are adsorbed by an adsorbent which is packed within the adsorption columns, whereby it is delivered as a refined gas after processed from said adsorption columns.

By using such an adsorbent in the method of the present invention, any process of prior art treatment for removing the moisture requiring any device for separating the moisture is not required. Thus the size of whole system can be minimized and the total cost of whole system reduced.

The adsorbent used is a carbon molecular sieve with approximately 0.3 nm (3Å angstrom) of pore diameter in average.

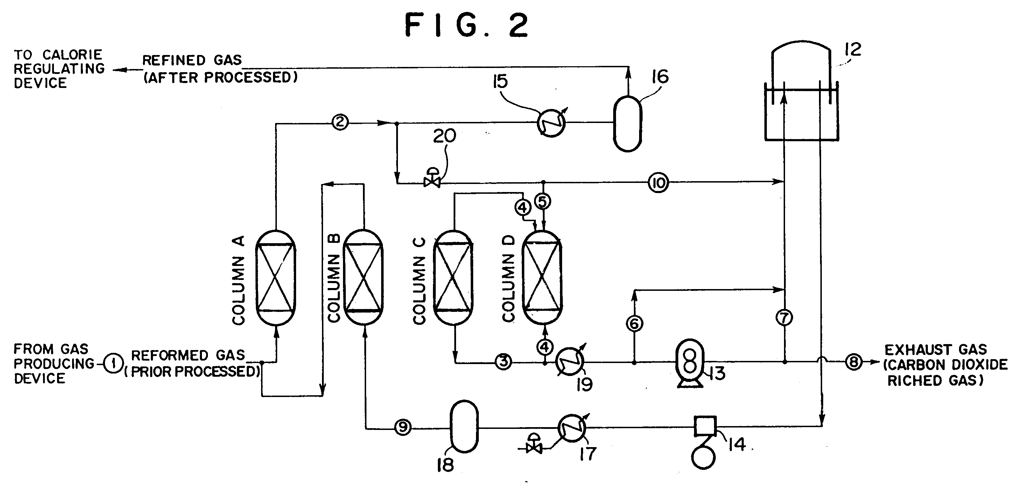

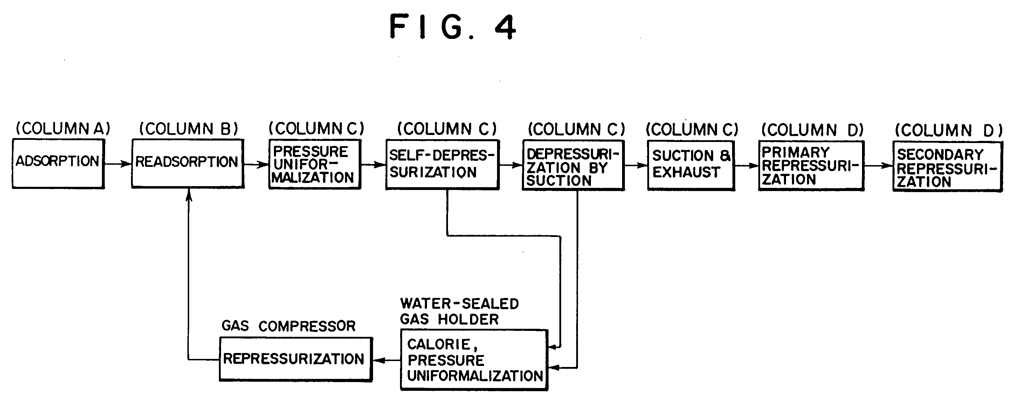

In the present invention, the above PSA device consists of four adsorption columns (A,B,C,D). Four cycles based on adsorption, readsorption, depressurization and repressurization are repeated by means of the four adsorption columns (A, B, C and D), a vacuum pump, the water-sealed gas holder and a compressor which are shown in the flow chart of Fig. 2 and the block diagram of process in Fig. 4, wherein when column A is in the adsorption process, column B is in the readsorption process, column C is in the pressure uniformalization and the self-depressurization, the depressurization by suction and the suction & exhaust and then the delivery of the evacuated carbon dioxide gas and lastly column D is in the primary repressurization (pressure uniformalization). In total 8 individual processes are carried out as shown in Fig. 4.

Note that those columns given in parenthesis in Fig. 4 show the working columns respectively when column A is in the state of refining (adsorption).

4

The following Table 1 shows the relationship between gas flow process and sequences when column A is in the refining (adsorption) process. The 8 individual processes may be summarized in the following five steps shown in the Table 1.

Table 1

In case of column A being in the refining (adsorption)

process.

```
-----------------------------------------------------------
                 Process           Gas flows according to each
                                   process.
-----------------------------------------------------------

            Refining (Adsorption):    ① - column A - ②

             Readsorption(recycle):  gas holder - compressor -

                                      ⑨ - column B - ①
    Step 1
            Primary repressuriz-    column C - ④ - column D
            ation(pressure unifor-
            marization):               ③ - ④


            Secondary repressur-    column A - ② - ⑤ -
            ization:                column D

-----------------------------------------------------------

            Refining (Adsorption):    ① - column A - ②

             Readsorption(recycle):  gas holder - compressor -

    Step 2                            ⑨ - column B - ①

            Secondary repressur-    column A - ② - ⑤ -
            ization:
                                    column D

            Self-depressurization:  column C - ③ - ⑤ -

                                    gas holder
```

5

| Process | Gas flows according to each process. |
|---|---|
| Refining (Adsorption): | (1) - column A - (2) |
| **Step 3** Readsorption(recycle): | gas holder - compressor - (9) - column B - (1) |
| Secondary repressur-ization: | column A - (2) - (5) - column D |
| Depressurization by suction: | column C - (3) - vacuum pump - (7) - gas holder |

| Process | Gas flows according to each process. |
|---|---|
| Refining (Adsorption): | (1) - column A - (2) |
| **Step 4** Readsorption(recycle): | gas holder - compressor - (9) - column B - (1) |
| Secondary repressur-ization: | column A - (2) - (5) - column D |
| Suction & exhaust (desorption): | column C - (3) - pump- (8) |

| Process | Gas flows according to each process. |
|---|---|
| Refining (Adsorption): | (1) - column A - (2) |
| Readsorption(recycle): | gas holder - compressor - (9) - column B - (1) |
| Rinsing: | column A - (2) - (10) - gas holder |
| **Step 5** Suction & exhaust (desorption): | column C- (3) -pump- (8) |
| Keep intact: | column D |

The above numbers show the gases in the state of each process under as follows and are also relevant to the figures especially figure 2:

(1) : Reformed gas (prior processed)
(2) : Refined gas (after processed)
(3) : Depressurized gases
(4) : Primary repressurization gases
(5) : Secondary repressurizaticn gases
(6) : Self-depressurized gases

⑦ : Evacuated gases
⑧ : Exhaust gas (desorbed carbon dioxide riched gas)
⑨ : Readsorption gases (recycle)
⑩: Rising gases

That is, the partial amount of refined gas delivered from the column A is used continuously for such two purposes as the secondary repressurization gas for column D and the rinsing gas for the recycle circuits so as to prevent fluctuation of the pressure and flow rate.

In the initial stage of pressure uniformalization in column C, an enriched combustible component is contained densely in the upper portion of column C and the depressurization to the intermediate pressure is performed through both outlets of the upper and lower portions at the same time and then the self-depressurized gas is flowed into column D through both inlets of column D as the primary repressurization gas.

Consequent to the above inflow in both directions, the recovery ratio of combustible gas and also the concentration of exhaust gas (i.e. $CO_2$) will be enhanced. Pursuantly, the depressurized gas with the intermediate pressure is transferred into the water-sealed gas holder and then when the inside pressure of column C is mostly balanced with the inside pressure of water-sealed gas holder, the gas of column C is delivered by a vacuum pump and depressed until around 66.65 kPa (500 Torr.) and sent into the water-sealed gas holder.

As described in the above, the purpose of accumulating those gases such as the depressurized gas with the intermediate pressure which still retains the combustible component and the evacuated gas sucked by the vacuum pump into the water-sealed gas holder with a uniformalizer will be to regulate the calorific value of desorption gas which fluctuates according to a difference in delivery time and furtherly to balance such pressures between delivery and suction pressures of a recycle gas compressor which will be described later.

The recycle gas with a constant pressure with mostly equalized calorific value is sent into column B by the recycle gas compressor because of an adsorption ability being retained still in column B wherein the carbon dioxide gas is readsorped and then returned to the reformed gas prior to processing. Further, in the last stage of the recycle process, the gas after processing is accumulated in the water-sealed gas holder partially. At the same time, another partial amount of gas after processing is utilized for rinsing the pipe line system whereby it enhances such the content of combustible components within the refined gas for a preparation of the next cycle. Thus, these series of steps are extremely effective to recover the combustible components without any bad influence upstream of the PSA device, furtherly to minimize the remainder of combustible components contained within the exhaust gas and moreover to stabilize those fluctuations of calorific value and pressure of refined gases downstream.

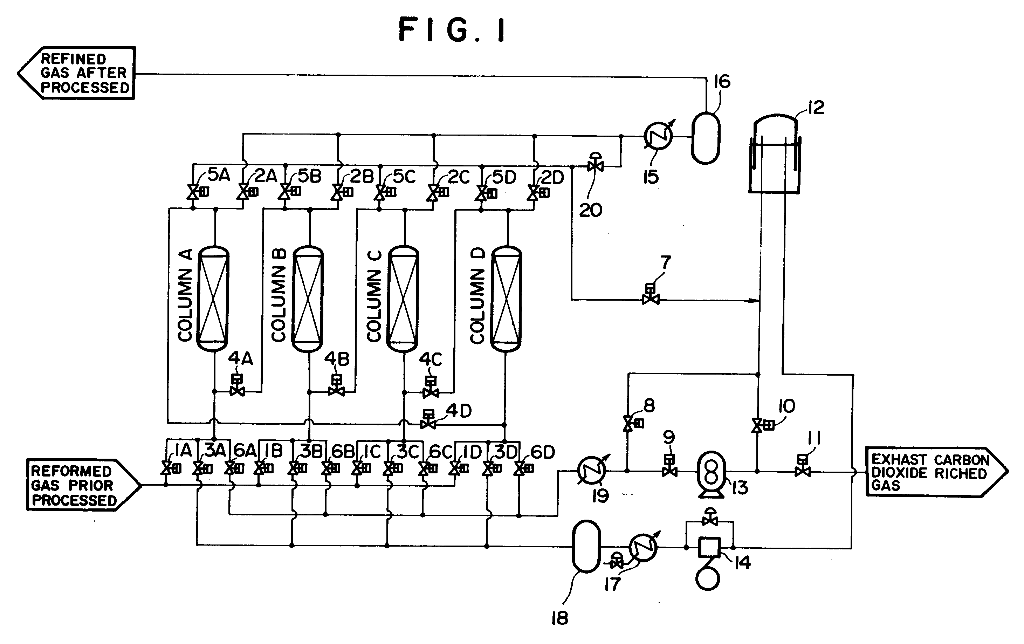

The present invention will now be described in detail with reference to Fig. 1 and Fig. 2. The method of this invention should be practiced preferably according to the device shown in Fig. 1. It comprises the four units of adsorption columns A, B, C and D; the water-sealed gas holder (12); the desorbed gas vacuum pump (13); the recycle gas compressor (14) and the other necessary devices which communicate each other through the pipe line system. According to the open-close action of valves being arranged at the necessary positions of pipe line such as (1A) - (1D), (2A) - (2D), (3A - 3D), (4A) - (4D), (5A) - (5D), (6A) - (6D), (7), (8), (9), (10) and (11), the gas flows are switched and controlled. Thus the eight processes involving four cycles with five steps in four units of adsorption column will be performed repeatedly and continuously. The operational sequences of the above adsorption columns A, B, C, and D are shown in Table 2 and the detail of valve operation is shown in Table 3 as follows;

EP 0 314 040 B1

Table 2　　　( detailed operations in each adsorption column )

| CYCLE | 1 | | | | | 2 | | | | | 3 | | | | | 4 | | | | |
|---|---|---|---|---|---|---|---|---|---|---|---|---|---|---|---|---|---|---|---|---|
| STEP | 1 | 2 | 3 | 4 | 5 | 1 | 2 | 3 | 4 | 5 | 1 | 2 | 3 | 4 | 5 | 1 | 2 | 3 | 4 | 5 |
| Column A | Refining ( adsorption ) | | | | | Readsorption ( recycle ) | | | | Rinsing | Pressure unifo- rmalization | Self-depressuri- zation | Depressurization by suction | Suction & exhaust | | Primary rep- ressurization | Secondary repressurization | | | Keep intact |
| Column B | Readsorption ( recycle ) | | | | Rinsing | Pressure unifo- rmalization | Self-depressuri- zation | Depressurization by suction | Suction & exhaust | | Primary rep- ressurization | Secondary repressurization | | | Keep intact | Refining ( adsorption ) | | | | |
| Column C | Pressure unifo- rmalization | Self-depressuri- zation | Depressurization by suction | Suction & exhaust | | Primary rep- ressurization | Secondary repressurization | | | Keep intact | Refining ( adsorption ) | | | | | Readsorption ( recycle ) | | | | Rinsing |
| Column D | Primary rep- ressurization | Secondary repressurization | | | Keep intact | Refining ( adsorption ) | | | | | Readsorption ( recycle ) | | | | Rinsing | Pressure unifo- rmalization | Self-depressuri- zation | Depressurization by suction | Suction & exhaust | |

Table 3 (Valves open-close positions in each step)

| STEP VALVES | 1 | 2 | 3 | 4 | 5 | 1 | 2 | 3 | 4 | 5 | 1 | 2 | 3 | 4 | 5 | 1 | 2 | 3 | 4 | 5 |
|---|---|---|---|---|---|---|---|---|---|---|---|---|---|---|---|---|---|---|---|---|
| 1A | O | O | O | O | O | X | X | X | X | X | X | X | X | X | X | X | X | X | X | X |
| 1B | X | X | X | X | X | X | X | X | X | X | X | X | X | X | X | O | O | O | O | O |
| 1C | X | X | X | X | X | X | X | X | X | X | O | O | O | O | O | X | X | X | X | X |
| 1D | X | X | X | X | X | O | O | O | O | O | X | X | X | X | X | X | X | X | X | X |
| 2A | O | O | O | O | O | X | X | X | X | X | X | X | X | X | X | X | X | X | X | X |
| 2B | X | X | X | X | X | X | X | X | X | X | X | X | X | X | X | O | O | O | O | O |
| 2C | X | X | X | X | X | X | X | X | X | X | O | O | O | O | O | X | X | X | X | X |
| 2D | X | X | X | X | X | O | O | O | O | O | X | X | X | X | X | X | X | X | X | X |
| 3A | X | X | X | X | X | O | O | O | O | O | X | X | X | X | X | X | X | X | X | X |
| 3B | O | O | O | O | O | X | X | X | X | X | X | X | X | X | X | X | X | X | X | X |
| 3C | X | X | X | X | X | X | X | X | X | X | X | X | X | X | X | O | O | O | O | O |
| 3D | X | X | X | X | X | X | X | X | X | X | O | O | O | O | O | X | X | X | X | X |
| 4A | O | O | O | O | O | X | X | X | X | X | X | X | X | X | X | X | X | X | X | X |
| 4B | X | X | X | X | X | X | X | X | X | X | X | X | X | X | X | O | O | O | O | O |
| 4C | X | X | X | X | X | X | X | X | X | X | O | O | O | O | O | X | X | X | X | X |
| 4D | X | X | X | X | X | O | O | O | O | O | X | X | X | X | X | X | X | X | X | X |
| 5A | X | X | X | X | X | X | X | X | X | X | O | X | X | X | X | O | O | O | O | X |
| 5B | X | X | X | X | X | O | X | X | X | X | O | O | O | O | X | X | X | X | X | X |
| 5C | O | X | X | X | X | O | O | O | O | X | X | X | X | X | X | X | X | X | X | X |
| 5D | O | O | O | O | X | X | X | X | X | X | X | X | X | X | X | O | X | X | X | X |
| 6A | X | X | X | X | X | X | X | X | X | X | O | O | O | O | O | O | X | X | X | X |
| 6B | X | X | X | X | X | O | O | O | O | O | O | X | X | X | X | X | X | X | X | X |
| 6C | O | O | O | O | O | O | X | X | X | X | X | X | X | X | X | X | X | X | X | X |
| 6D | O | X | X | X | X | X | X | X | X | X | X | X | X | X | X | O | O | O | O | O |
| 7 | X | X | X | X | O | X | X | X | X | O | X | X | X | X | O | X | X | X | X | O |
| 8 | X | O | X | X | X | X | O | X | X | X | X | O | X | X | X | X | O | X | X | X |
| 9 | X | X | O | O | O | X | X | O | O | O | X | X | O | O | O | X | X | O | O | O |
| 10 | X | X | O | X | X | X | X | O | X | X | X | X | O | X | X | X | X | O | X | X |
| 11 | X | X | X | O | O | X | X | X | O | O | X | X | X | O | O | X | X | X | O | O |

O: valve opened    X: valve closed

The step in which the adsorption column A is in the process of refining according to Fig. 2 and Fig. 4 will now be described.

As shown in Figs. 2 and 4, when the column A is in the refining, column B is in the readsorption (recycle), column C is operated by the series or pressure uniformalization, self-depressurization, depressurization by suction, suction & exhaust in turn, further, column D is in the primary repressurization (pressure uniformalization) and then secondary repressurization respectively.

Description will be made to 1 cycle in the adsorption column A.

(1) Adsorption Step (a)

The produced gas before processing (1) is passed by way of the valve 1A and introduced together with the gas from the valve 4A into the adsorption column A and then taken out as a refined gas (2) from the exit of the column after adsorbing $CO_2$ and moisture to the adsorbent filled therein. The refined gas (2) is sent by way of the valve 2A and supplied continuously to a refined gas cooler 15 and a refined gas surge tank 16 and then flows to the calorie conditioning device at the downstream to the PSA process. A portion of the thus obtained refined gas is used as a secondary pressurization gas or a substituting rinsing gas for the pipe lines.

### (2) Readsorption Step (b)

A recycle gas (9) which is reserved in the water-sealed gas holder 12 (desorbed gas containing $CH_4$ component as described in the steps (d), (e) later) is pressurized by the recycle gas compressor 14, introduced by way of the recycle gas cooler 17 and a recycle gas moisture separator 18 and through the valve 3A in a parallel current into the adsorption column A wherein $CO_2$ is readsorbed. The gas from the exit of the column is joined through the valve 4D with the reformed gas prior to processing (1) and supplied to the column in other adsorption step. Since the temperature of the recycle gas (9) is elevated by the pressurization, it is cooled to the adsorption temperature in the recycle gas cooler 17. In the last stage of the readsorption step, the gas temperature is again elevated by bypassing the recycle gas cooler 17 or reducing the flow rate of the cooling water into the recycle gas cooler 17 for the desorption starting from the succeeding step. Accordingly, since the adsorption temperature can be lowered while the desorption temperature can be elevated, the adsorption and desorption conditions becomes extremely advantageous.

### (3) Pressure Uniformalization Step (c)

Column A and another column for which the step (f) to be described later has been completed and in which the pressure is lowest are brought into communication to each other, through the upper and the lower portions to recover the pressure, as well as recover the $CH_4$ component. That is, the primary pressurization gas (4) is sent from the upper portion of the column through the valve 5A and the valve 5B into the column B, while the primary pressurization gas (3) is sent from the lower portion of the column through the valve 6A and by way of the valve 6B to the column B, thereby balancing the pressure between the column A and column B. Thus, the pressure in both of the columns is made uniform. The primary pressurization gas (4) sent from the upper portion of the column contains the $CH_4$ ingredient at high concentration. In this way, pressure normalization is conducted both in the upper and the lower directions of the adsorption columns.

### (4) Self Depressurization Step (d)

Then, the pressure in the adsorption column is depressurized in a counter current manner by the self-pressure to the pressure of the water-sealed gas holder 12, that is, to about 103 kPa (200 mmAq). A self depressurization gas (6) containing the $CH_4$ ingredient is sent by way of the valve 6A, desorption gas cooler 19 and the valve 8 to the water-sealed gas holder 12.

### (5) Vacuum Depressurization Step (e)

Successively, the pressure in the adsorption column is reduced in a counter current manner from the pressure in the water-sealed gas holder 12 to an intermediate pressure of about 67 kPa (500 Torr) by the desorbed gas vacuum pump 13.

The vacuum depressurization gas (7) containing the $CH_4$ component is sent by way of the valve 6A of the desorbed cooler 19, the valve 9, the desorbed gas vacuum pump 13 and the valve 10 to the water-sealed gas holder 12. When the $CH_4$ component is reduced in the desorbed gas while leaving $CO_2$ at high purity, the flow channel is switched for the recovery of $CO_2$ and moisture in the succeeding step.

### (6) Vacuum Desorption Step (f)

The desorbed gas is further depressurized from the intermediate pressure to about 6.7 kPa (50 Torr) by the desorbed gas vacuum pump 13 in a counter current manner to desorb $CO_2$ and the moisture. The valve 10 is closed and, instead, the valve 11 is opened, and $CO_2$ and moisture are taken out as the vacuum desorbed gas (8).

In the regeneration of the adsorbent by the desorbed gas vacuum pump 13, the amount of the exhaust gas is reduced as the vacuum degree is increased in view of the exhaustion characteristics of the pump 13.

Further, also the component in the exhaust gas, $CO_2$ is increased along with the increase of the degree of vacuum and, also the ratio for $CO_2/CH_4$ is also increased.

EP 0 314 040 B1

(7) Primary Pressurization step (g)

A primary pressurization gas (4) is received from the upper portion of the adsorption column D under the step (c) through the valve 5A in a counter current manner into the column, while the primary pressurization gas (3) is received from the lower portion of the column in a parallel current into the column. In this way, in the primary pressurization, the pressure in the column is elevated to a balanced pressure by receiving the uniform pressure gas both in the upper and the lower directions of the column.

Meanwhile, a portion of the refined gas (2) from the upper portion of the column is received as the secondary pressurization gas (5) by way of a constant flow rate control valve 20 through the valve 5A into the column. That is, in this step, the primary pressurization and the secondary pressurization are conducted in parallel.

(8) Secondary Pressurization Step (h)

A portion of the refined gas (2) is received in a counter current manner as the secondary pressurization gas (5) by way of the flow rate control valve 20 passing the valve 5A into the upper portion of the column to elevate the pressure to the adsorption pressure. In this case, in order that the pressure and the flow rate of the refined gas (2) does not fluctuate, the constant flow rate control valve 20 is disposed to the pipe lines for taking out the refined gas (2) and the secondary pressurization gas (5) is supplied at a constant flow rate.

(9) Stand-by Step (i)

When the secondary pressurization with the refined gas (2) is completed and the pressure in the column reaches the adsorption pressure, the adsorption column is in a stand-by state under the pressure. This is not always necessary but a selective means. Meanwhile, a portion of the refined gas (2) is sent as a rinsing-gas (10) by way of the constant flow rate control valve 20 through the valve 7 into the water-sealed gas holder 12 and the recycle gas pipe line.

Substitution with the refined gas for the recycle gas circuit is effective for enhancing the content of the combustible gases in the refined gas after processing in the succeeding step.

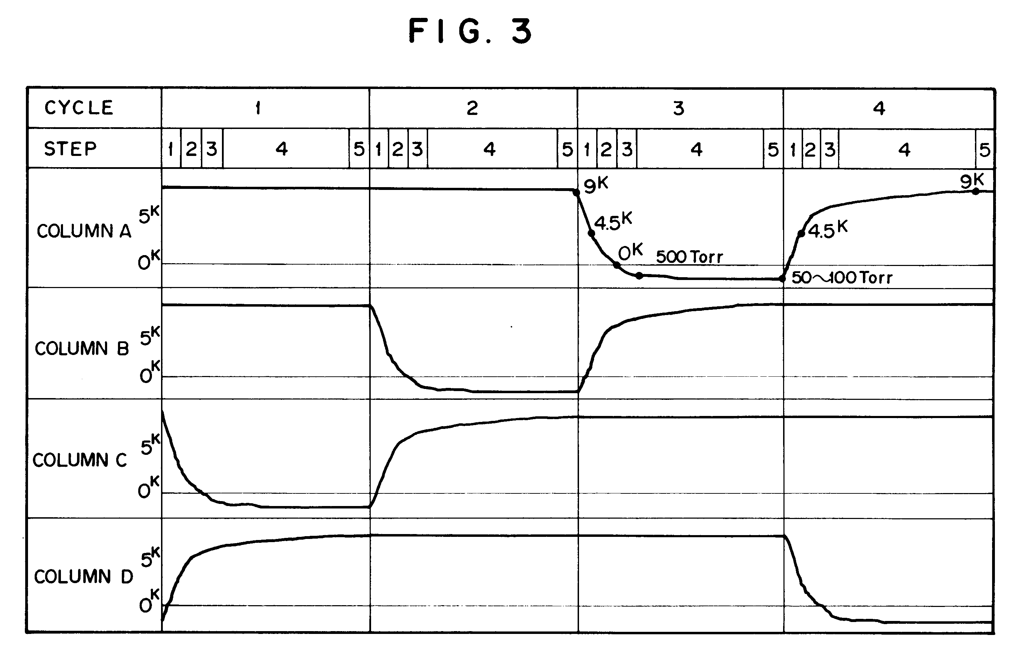

The pressure variation in the adsorption column in each of the steps will be shown in Fig. 3. As can be seen from the figure, the pressure in the column in the adsorption step connected to the main stream line of the process is equal with the pressure in other two columns, so that there is no pressure shock at all upon switching the columns. That is, switching between the adsorption columns connected to the main stream dine of the process is conducted under the identical adsorption pressure for each of the three columns.

Further, the results of measurement in the examples for the gas flow rate and calorific values of the gas in each of the sections in the above-mentioned series of steps are as shown in Table 4.

Table 4

| Components | Reformed gas prior processed (1) | Recycle gas (9) | Refined gas after processed (2) | Exhaust gas (carbon dioxide riched gas) (8) |
|---|---|---|---|---|
| $H_2$ | 2.40 | 1.10 | 3.17 | - |
| CO | 0.10 | 0.04 | 0.13 | - |
| $CO_2$ | 25.00 | 50.06 | 1.37 | 99.00 |
| $CH_4$ | 72.50 | 48.80 | 95.33 | 1.00 |
| Flow ratio | 100.00 | 18.80 | 75.80 | 24.20 |
| Calorific value | 6.991 | 4.689 | 9.192 | 95 |
| (Remarks) Components (%), Flow ratio (%), Calorific value (kcal/Nm$^3$) | | | | |

As shown in the above Table 4, it is obvious that the present invention enables to provide to recover the combustible components with an extremely high yield from the feed gas which is produced by PSA device conventionally, whereby the combustible component is scarcely contained anything in the exhaust gas, to which is the key of city gas production. It is seen that only 1% of combustible component (i.e. $CH_4$) is

11

exhausted in Table 4.

As to the efficiency of adsorbent in the present invention, since aforesaid carbon molecular sieves with approximately 0.3 nm (3Å angstrom) of pore diameter in average is adopted wherein can adsorb $CO_2$ gas and $H_2O$ moisture simultaneously so that this adsorbent has an uncomparably high efficiency and productivity. Besides, as to the relationship between the partial pressure owned by $CO_2$ gas and $CH_4$ gas and the adsorption properties of adsorbent, basically $CO_2$ gas has a higher partial pressure than $CH_4$ gas, therefore, in the highly pressurized environment such as in the refining process with 0,88 MPa (9 kg/cm$^2$ G), the adsorption of $CO_2$ gas by the adsorbent is preferably effective due to its high partial pressure, in contrast the lower partial pressure owned $CH_4$ gas is not adsorbed in that environment. The above relationship between the both gases earns a prominent effect upon the adsorbent. Because, it gains 75.80% of refined gas ② and only 18.80% of recycle gas is returned, whereby the whole size of facilities can be minimized due to the minimized flow ratio of recycle gas.

Since the present invention is comprised as the above, it has the following technical effects;

(1) Since a desorbed gas is recycled firstly and then during the recycle the desorbed gas is further readsorbed repeatedly, it can recover a combustible components for city gas with an extremely high yield from an exhaust gas which is produced by PSA method, wherein it can minimize the loss of combustible gas from the exhaust gas. As the result, the refined gas becomes to contain a high calorie contents for the city gas, in contrast the exhaust gas contains an extremely lower content of the combustible component, thus such expenses of treatment against the exhaust gas air pollution can be save due to its no pollution character.

In the adsorption columns, the recycle gas is readsorbed through such exclusive type adsorption columns, thus the recycle gas rate can be minimized whereby its total size of production facility and also its cost are minimized.

(2) The main problems which PSA method involves are that it involves fluctuations upon the gas flow rate, gas pressure and its calorie as first and secondly it involves a high ratio of loss upon the combustible components within the exhaust gas. In the connection, the advantages and technical effects which the present invention has been achieved are that:

a) the recycle gas is reserved in a water-sealed gas holder once time.

b) the recycle gas is returned to a reformed gas prior processed in the adsorption columns,

c) a partial amount of refined gas is taken out through a constant gas flow rate controlling valve wherein used for repressurizing the columns and rinsing the pipe lines,

d) whereby the fluctuations upon the gas flow rate, pressure and its calorie which are involved by device have been solved,

e) furthermore the problems of PSA device in upstream and downstream such as a gas producing device and a calorie regulating device have been solved without any fluctuation upon the calorie for the city gas.

## Claims

1. A method for continuously removing carbon dioxide gas and water moisture from a reformed gas produced in the process of city gas production; the method comprising a pressure swing adsorption process utilizing at least four adsorption columns containing carbon molecular sieves with an average pore diameter of 0.3 nm (3Å) as adsorbent, each of which successively undergoing in a cyclical manner the steps of:

(a) feeding at an elevated pressure the wet reformed gas without any water content reducing pretreatment into the inlet of the adsorption column, adsorbing carbon dioxide and water from gas and withdrawing refined gas from the outlet of the adsorption column;

(b) readsorbing at elevated pressure carbon dioxide from a gas stream which is concurrently fed from a water-sealed gas holder into the adsorption column and recycling the gas which is withdrawn from the outlet of the adsorption column during this step to the wet reformed gas feed stream;

(c) equalizing the gas pressure between said adsorption column and a parallel column which has terminated the evacuation step (f) by bringing in fluid communication respectively the inlets and the outlets of both columns;

(d) countercurrently further depressurizing the adsorption column by bringing its inlet in fluid communication to the water sealed gas holder, wherein the pressure of this gas is regulated;

(e) further countercurrently depressurizing the adsorption column by mechanical suction and introducing the gas which is sucked off into the water-sealed gas holder, as well;

(f) further countercurrently depressurizing the adsorption column by mechanical suction to desorb and extract carbon dioxide and water from the adsorbent and discarding the gas which is sucked off;

(g) primarily repressurizing said adsorption column by equalizing the gas pressure between said adsorption column and another column which has terminated the readsorption step (b) and bringing therefor in fluid communication respectively the inlets and the outlet of both columns and simultaneously secondarily repressurizing said adsorption column by countercurrently introducing a partial amount of the refined gas stream via a constant flow rate controlling valve; and

(h) further secondarily repressurizing said adsorption column towards the pressure of the adsorption step (a) by countercurrently introducing a partial amount of the refined gas stream via a constant flow rate controlling valve.

2.  A method as set forth in claim 1, wherein the pressure of said desorbed gas reserved in said water-sealed gas holder is approximately at atmospheric pressure.

3.  A method as set forth in claim 1, wherein an intermediate pressure of said depressurization column under the steps (e) is 66.65 kPa (500 Torr.)

4.  A method as set forth in claim 1, wherein a partial amount of said refined gas through a constant flow rate controlling valve under the step (h) may be used to rinse the pipelines of the system.

5.  A method as set forth in claim 1, wherein said refined gas under the step (a) contains mainly $CH_4$ with $H_2 CO_2$ and CO, and said desorbed gas under the steps (d) and (e) contains mainly $CO_2$ with $CH_4$.

6.  A method as set forth in claim 1, wherein said reformed gas prior processed is produced from reforming such LPG, methanol and naphtha with steam wherein said reformed gas consists of combustible components such as $CH_4$, $H_2$ and the like mainly.

**Patentansprüche**

1.  Verfahren zum kontinuierlichen Entfernen von Kohlendioxid-Gas und Wasserfeuchtigkeit aus einem beim Stadtgaserzeugungsprozeß erzeugten Spaltgas; wobei das Verfahren einen Druckschwankungs-Adsorptionsprozeß umfaßt, der mindestens vier Adsorptionskolonnen benutzt, welche als Adsorptionsmittel Kohlenstoff-Molekularsiebe mit einem mittleren Porendurchmesser von 0,3 nm (3Å) enthalten, von denen jede in einer zyklischen Weise nacheinander die Schritte durchläuft:

(a) Einspeisen des nassen Spaltgases ohne jegliche Vorbehandlung zur Reduzierung des Wassergehaltes unter einem erhöhten Druck in den Einlaß der Adsorptionskolonne, Adsorbieren von Kohlendioxid und Wasser aus Gas und Abziehen von veredeltem Gas aus dem Auslaß der Adsorptionskolonne;

(b) Erneutes Adsorbieren (Readsorbieren) von Kohlendioxid unter erhöhtem Druck aus einem Gasstrom, der gleichzeitig aus einem Gasbehälter mit Wasserverschluß in die Adsorptionskolonne eingespeist wird, und Zurückführen des während dieses Schritts aus dem Auslaß der Adsorptionskolonne abgezogenen Gases zum nassen Spaltgas-Speisestrom;

(c) Ausgleichen des Gasdrucks zwischen der besagten Adsorptionskolonne und einer parallelen Kolonne, welche den Evakuierungsschritt (f) beendet hat, indem man jeweils die Einlässe und die Auslässe beider Kolonnen in Fluidverbindung bringt;

(d) weiteres Abbauen des Drucks in der Adsorptionskolonne im Gegenstrom, indem man ihren Einlaß mit dem Gasbehälter mit Wasserverschluß in Fluidverbindung bringt, in welchem der Druck dieses Gases geregelt wird;

(e) weiteres Abbauen des Drucks in der Adsorptionskolonne im Gegenstrom durch mechanisches Absaugen und zuführen des abgesaugten Gases ebenfalls in den Gasbehälter mit Wasserverschluß;

(f) weiteres Abbauen des Drucks in der Adsorptionskolonne im Gegenstrom durch mechanisches Absaugen zum Desorbieren und Extrahieren von Kohlendioxid und Wasser aus dem Adsorptionsmittel und Abstoßen des abgesaugten Gases;

(g) primär Wiederbeaufschlagen der besagten Adsorptionskolonne mit Druck durch Ausgleichen des Gasdrucks zwischen der besagten Adsorptionskolonne und einer anderen Kolonne, welche den Readsorptionsschritt (b) beendet hat, wobei dafür jeweils die Einlässe und der Auslaß beider Kolonnen in Fluidverbindung gebracht werden, und gleichzeitig sekundär Wiederbeaufschlagen der besagten Adsorptionskolonne mit Druck durch Zufuhr einer Teilmenge des Stroms des veredelten Gases im Gegenstrom über ein Konstantstrommengen-Steuerventil; und

(h) weiter sekundär Wiederbeaufschlagen der besagten Adsorptionskolonne mit Druck in Richtung des Drucks des Adsorptionsschritts (a) durch Zuführen einer Teilmenge des Stroms des veredelten Gases im Gegenstrom über ein Konstantstrommengen-Steuerventil.

2. Verfahren nach Anspruch 1, dadurch gekennzeichnet, daß der Druck des besagten, in dem besagten Gasbehälter mit Wasserverschluß zurückbehaltenen desorbierten Gases ungefähr bei Atmosphärendruck liegt.

3. Verfahren nach Anspruch 1, dadurch gekennzeichnet, daß ein Zwischendruck der besagten Druckabbau-Kolonne während der Schritte (e) 66,65 kPa (500 Torr) beträgt.

4. Verfahren nach Anspruch 1, bei welchem eine Teilmenge des besagten veredelten Gases durch ein Konstantstrommengen-Steuerventil während des Schritts (h) verwendet werden kann, um die Leitungen des Systems zu spülen.

5. Verfahren nach Anspruch 1, dadurch gekennzeichnet, daß das besagte veredelte Gas während des Schritts (a) hauptsächlich $CH_4$ mit $H_2$, $CO_2$ und CO enthält, und das besagte desorbierte Gas während der Schritte (d) und (e) hauptsächlich $CO_2$ mit $CH_4$ enthält.

6. Verfahren nach Anspruch 1, dadurch gekennzeichnet, daß das besagte noch nicht behandelte Spaltgas aus einer Reformierung von beispielsweise Flüssiggas, Methanol und Naphta mit Dampf erzeugt wird, wobei das besagte Spaltgas hauptsächlich aus brennbaren Bestandteilen, wie beispielsweise $CH_4$, $H_2$ und dergleichen besteht.

**Revendications**

1. Procédé d'élimination en continu de dioxyde de carbone gazeux et d'humidité à partir d'un gaz reformé produit par un procédé de production de gaz de ville, le procédé comprenant un procédé d'adsorption par variation de pression utilisant au moins 4 colonnes d'adsorption remplies d'un tamis moléculaire ayant des pores d'un diamètre moyen égal à 0,3 nm (3Å) comme adsorbant, chacune d'elles subissant d'une manière cyclique les étapes consistant

(a) à alimenter sous pression la colonne d'adsorption avec le gaz reformé humide sans un quelconque traitement préalable réduisant sa teneur en eau, à réaliser l'adsorption du dioxyde de carbone et de l'eau à partir du gaz et à soutirer le gaz raffiné en sortie de la colonne d'adsorption,

(b) à réadsorber sur la colonne sous une pression élevée du dioxyde de carbone à partir d'un flux de gaz provenant d'une bonbonne de gaz à l'abri de l'humidité et alimentant simultanément la colonne, à recycler le gaz sortant de la colonne d'adsorption pendant cette étape vers le flux gazeux humide reformé.

(c) à égaliser les pressions gazeuses dans ladite colonne d'adsorption et dans une colonne parallèle dans laquelle l'étape d'évacuation (f) est terminée, en établissant la communication des fluides sortant et entrant les deux colonnes,

(d) à dépressuriser encore plus la colonne dans le sens contraire du courant en faisant communiquer son entrée avec la bonbonne de gaz à l'abri de l'humidité, régulant ainsi la pression de ce gaz,

(e) à dépressuriser encore plus la colonne d'adsorption dans le sens contraire du courant par aspiration mécanique et à envoyer le gaz éliminé par aspiration vers la bonbonne de gaz à l'abir de l'humidité, et

(f) à dépressuriser encore plus dans le sens contraire du courant la colonne d'adsorption par aspiration mécanique afin de désorber et d'extraire le dioxyde de carbone et l'eau de l'adsorbant et d'éliminer ce gaz retiré par aspiration,

(g) à remettre ladite colonne d'adsorption sous une pression primaire en égalisant les pressions gazeuses dans ladite colonne d'adsorption et dans une autre colonne dans laquelle l'étape de réadsorption (b) est terminée, et à établir ainsi la communication des fluides respectivement entre l'entrée et la sortie des deux colonnes et à remettre simultanément sous pression secondaire ladite colonne d'adsorption en introduisant dans le sens contraire du courant une quantité partielle du flux de gaz raffiné via une vanne permettant d'assurer un débit constant, et

(h) à remettre ladite colonne d'adsorption à la pression de l'étape d'adsorption (a) en introduisant dans le sens contraire du courant une quantité partielle du flux gazeux raffiné via une vanne permettant d'assurer un débit constant.

2. Procédé conforme à la revendication 1 dans lequel la pression dudit gaz désorbé stocké dans ladite bonbonne de gaz à l'abri de l'humidité est environ égale à la pression atmosphérique.

3. Procédé conforme à la revendication 1 dans lequel la pression intermédiaire de ladite colonne de dépressurisation de l'étape (e) est égale à 66,65 kPa (500 torr).

4. Procédé conforme à la revendication 1 dans lequel une partie dudit gaz raffiné soutirée par une vanne permettant d'assurer un débit constant de l'étape (h) peut être utilisé pour rincer les conduits du système.

5. Procédé conforme à la revendication 1 dans lequel ledit gaz raffiné de l'étape (a) contient principalement du $CH_4$ ensemble avec du $H_2 CO_2$ et CO, et ledit gaz désorbé des étapes (d) et (e) contient principalement du dioxyde de carbone et du méthane.

6. Procédé conforme à la revendication 1 dans lequel ledit gaz reformé avant traitement est produit par craquage de GPL, de méthanol et de naphta avec de la vapeur et dans lequel ledit gaz reformé est constitué principalement de composants combustibles, tels que le méthane et l'hydrogène et des composés semblables.

# FIG. 1

REFINED GAS AFTER PROCESSED

REFORMED GAS PRIOR PROCESSED

EXHAST CARBON DIOXIDE RICHED GAS

COLUMN A  COLUMN B  COLUMN C  COLUMN D

EP 0 314 040 B1

# F I G. 2

TO CALORIE
REGULATING ◄── REFINED GAS
DEVICE           (AFTER PROCESSED)

15    16

2    20

10

4  5

COLUMN A   COLUMN B   COLUMN C   COLUMN D

12

FROM GAS
PRODUCING ─① REFORMED GAS
DEVICE        (PRIOR PROCESSED)

3    4

6    7

8 ──► EXHAUST GAS
     (CARBON DIOXIDE
     RICHED GAS)

19    13

9    18    17    14

EP 0 314 040 B1

# FIG. 3

| CYCLE | 1 | 2 | 3 | 4 |
|---|---|---|---|---|

Column A: 9K, 5K, 4.5K, 0K, 500 Torr, 50~100 Torr, 9K, 4.5K

Column B: 5K, 0K

Column C: 5K, 0K

Column D: 5K, 0K

F I G. 4